# EUROPEAN PATENT APPLICATION

(11) **EP 2 711 706 A1**
(43) Date of publication of application: **26.03.2014**
(21) Application number: 12185371.7
(22) Date of filing: 21.09.2012
(51) Int. Cl.: G01N 33/569

(54) **Mycobacterial thiolperoxidase and its use**

(71) Applicant: LIONEX Diagnostics and Therapeutics GmbH, 38126 Braunschweig (DE)
(72) Inventor: Singh, Mahavir Prof. Dr., 38124 Braunschweig (DE); Baumann, Ralf Dr, 40225 Düsseldorf (DE); Kämpfer, Susanne, 38159 Vechelde (DE); Spallek, Ralf Dr, 30459 Hannover (DE); Oehlmann, Wulf Dr, 30177 Hannover (DE)
(74) Representative: Kröncke, Rolf

(57) **Abstract**

In the first aspect, the present invention relates to mycobacterial thiolperoxidase for use in diagnosing or determining tuberculosis infection in an individual. In a further aspect, methods are provided for diagnosing or determining the status of tuberculosis infection in individuals infected or suspected to be afflicted with tuberculosis infection comprising the step of determining the level or amount of antibodies against mycobacterial thioperoxidase in a sample of an individual. Moreover, methods are provided for the stratification of the treatment regimen of said individual as well as for monitoring the change from latent to active status of tuberculosis infection. Moreover, the present invention provides kits for use in diagnosing or detecting tuberculosis infection as well as the status of the same based on the level or amount of mycobacterial thiolperoxidase.

## Description

In the first aspect, the present invention relates to mycobacterial thiolperoxidase for use in diagnosing or determining tuberculosis infection in an individual. In a further aspect, methods are provided for diagnosing or determining the status of tuberculosis infection in individuals infected or suspected to be afflicted with tuberculosis infection comprising the step of determining the level or amount of antibodies against mycobacterial thioperoxidase in a sample of an individual. Moreover, methods are provided for the stratification of the treatment regimen of said individual as well as for monitoring the change from latent to active status of tuberculosis infection. Moreover, the present invention provides kits for use in diagnosing or detecting tuberculosis infection as well as the status of the same based on the level or amount of mycobacterial thiolperoxidase.

### Prior art

Approximately one third of the world's population has latent infection with mycobacterium tuberculosis. Each day around 25.000 people develop active TB and more than 4.500 die of the disease according to the WHO. This emphasizes the need for early identification and effective treatment of this basically curable disease.

Currently approaches to diagnose active TB continue to rely on initial clinical suspicion and subsequent confirmation by sputum microscopy and culture. However, these tests have major drawbacks. Sputum smear microscopy has a low sensitivity and sputum culture is technically sophisticated and takes several weeks to give a reliable result. However, diagnosis demands for rapid results which may be obtained by liquid culture with MGIT (mycobacteria growth indicator tube) but culture facilities are not readily available especially in most high burden/resource limited settings. Other tools for diagnosing TB includes tuberculin skin tests (TST) or the more expensive and complex interferon gamma release assays (IGRA). However, these culture based techniques are limited in use due to laborious technical as well as cost expensive work.

Serodiagnosis tests for the detection of antibodies against defined recombinant antigens from *M.turberculosis* of TB patients have been widely tested. However, the first generation of the diagnosic tests were characterized in low specificity which improved by using highly purified recombinant antigens. Further, there are still existing strong variations in antibody response in different TB diseased individuals and community controls in the same or different regions of the world are probably caused by genetically and immunologically diverse individuals and populations, variation of infecting *M.tuberculosis* strains expressing partially different antigens or a high proportion of LTBI (latent TB infection) control subjects. Therefore, the search for more ideal antigens and antigen combinations is continued in order to enable specific identification of most TB patients also, even in endemic settings. Antibody based assays have the advantage of being robust, cost-effective and simple in use, thus, ideal for use in a field setting and in particular, for use in low-income countries.

However, the serodiagnostic tests on the market e.g. are not in a position to differentiate between latent and active status of tuberculosis infection.

Moreover and quite important, no means or tools are available for determining efficiency and efficacy of the treatment regimen against TB. That is, during DOTS (directly observed treatment short course) consisting of a four-drug intensive phase of two months, followed by a two-drug continuation phase of four months (e.g. Luna, JAC, Tuberculosis guide for specialist physicians. Paris:

International Union against tuberculosis and ly disease, 2004).

However, the time period before obtaining results on treatment efficacy is very long and, consequently, changes in the treatment regimen is not possible during the treatment. In particular, it is desirable to be in a position of differentiating between the different types of responders during treatment, namely slow and fast responders, respectively. It is of particular interest to allow differentiation of the type of response prospectively. Slow responders are those patients who show treatment success in longer than three months duration while fast responders are individuals showing treatment success within 3 months.

Hence, there is a need for further compounds suitable for use in diagnosing or determining tuberculosis infection in an individual. Said compounds or antibodies against said compounds may be used in methods allowing to diagnose or differentiate tuberculosis infection. Accordingly, there is a need in the art for approaches for detection and determination of the therapeutic regimen, or, for predicting the clinical outcome or determining the treatment course as well as determining the status of tuberculosis infection in an individual suffering therefrom. For effective development of drugs for TB, there is a great need of methods and procedures for detecting those individuals who respond slowly to current drugs.

The present invention aims in providing new suitable tools or means, like compounds for use in diagnosing or determining tuberculosis infection in an individual.

### Summary of the present invention

In a first aspect, the present invention relates to a mycobacterial thiolperoxidase, in particular, thiolperoxidase (TPX) of *mycobacterium tuberculosis (M.tuberculosis)*, for use in diagnosing or determining tuberculosis infection (TB) in an individual.

In a further aspect, the present invention relates to the use of a mycobacterial thiolperoxidase in the differentiation between latent tuberculosis infection and active tuberculosis infection. Moreover, tpx allows to differentiate between individuals being slow treatment responders and fast treatment responders, in particular, during the intensive phase of TB treatment.

In another aspect of the present invention, a method for diagnosing or determining the status of tuberculosis infection in an individual afflicted with or suspected to be afflicted with a tuberculosis infection is provided. The method is based on the determination of a level or amount of antibodies against tpx in a sample of an individual and determining or diagnosing the status of said individual. Further, a method for the stratification of therapeutic regimen in an individual afflicted with tuberculosis infection is provided based on determining the level or amount of antibodies against tpx. Moreover, a method for the clinical outcome or determining the treatment course of tuberculosis infection is provided based on determining the level or amount of antibodies against tpx. Moreover, a method for monitoring the change from latent to active status of tuberculosis infection based on a level or amount of antibodies against tpx is provided.

Furthermore, the present invention relates to the use of a kit for diagnosing or determining the status of tuberculosis infection, for predicting a clinical outcome or determining the treatment course in an individual infected or suspected to be afflicted with tuberculosis infection, or for the stratification of the therapeutic regimen of an individual with tuberculosis infection or monitoring the progression of tuberculosis infection, or for determining the transition from latent to active status of tuberculosis infection in an individual, said kit comprises means for determining the level or amount of antibodies against mycobacterial thiolperoxidase. Finally, an assay for conducting the methods according to the present invention is provided.

That is, the present inventors recognized that the mycobacterial thiolperoxidase, in particular, the mycobacterial tuberculosis thiolperoxidase represents a suitable means for use in diagnosing or determining tuberculosis infection as well as differentiating the status of tuberculosis infection, accordingly. Particular, in serodiagnosis antibodies against tpx demonstrate promising accuracy, i.e. high specificity and sensitivity compared to compounds known in the art. Hence, it is possible to differentiate at a very early time point during treatment or even prospectively between individuals responding slow or fast on the treatment regimen.

Furthermore, tpx is suitable as an antigen in cell based tests, like cytokine release assays, e.g. IGRA or IL-2 based release assays. These cell based assays and suitable methods allowing for diagnosing or detecting the status of tuberculosis infection in an individual afflicted with or suspected to be afflicted with tuberculosis infection, as well as for the stratification of the therapeutic regimen, determining the treatment course or predicting the clinical outcome. In addition, suitable kits for use in the cell based assays using tpx as antigen for stimulation of said cells are provided.

### Brief description of the drawings

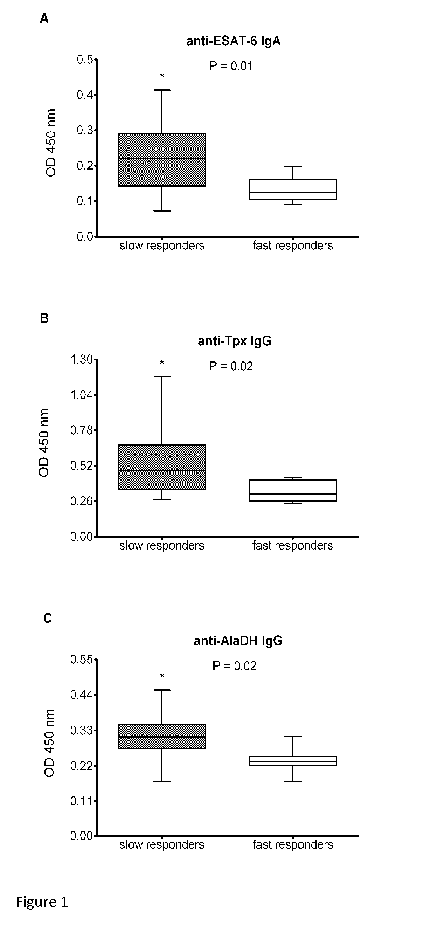

Figure 1: Figure 1 shows Box- and whisther plots showing the optical density (OD) values of the anti-ESAT-6 IgA, anti-Tpx IgG and anti-AlaDH IgG serodiagnosis assays for the comparison of eight fast and 13 slow intensive anti-TB treatment responders (P=0.01; P=0.02 and P=0.02, respectively).

### Detailed description of the present invention

The present invention relates in a first aspect to mycobacterial thiolperoxidase (tpx), in particular, thiolperoxidase of *Mycobacterium tuberculosis,* for use in diagnosing or determining tuberculosis infection in an individual.

It has been recognized by the present inventors that tpx represents a suitable tool in serodiagnosis of tuberculosis infection. Moreover, tpx allows serodiagnosis of latent tuberculosis infection (LTBI). Further, the serodiagnostic marker is particularly useful for in endemic settings. Moreover, tpx may be used as antigen in cell culture based tests, like IGRAs or in tuberculin skin tests.

As used herein, the term "individual" in the context of the present invention is typically a mammal, in particular, a mammal as a human, a non-human primate or other mammals susceptible for mycobacterial infection. The individual can be a male or female. The individual can be one that has been previously diagnosed with or identified as suffering from tuberculosis infection and, optionally, but need not to have already undergone treatment for tuberculosis infection. An individual can also be one who has been diagnosed with or identified as suffering from tuberculosis infection but who has shown improvement in the disease as a result of receiving one or more treatments for the infection, accordingly. Moreover, the individual may also be one who has not been previously diagnosed or identified as having tuberculosis infection.

The term "determining" or "detecting" as used herein refers to assessing the presence, absence, quantity, level or amount of either a given substance within a clinical or subject derived sample, including quality and/or quantitative concentration levels of substances otherwise evaluating values or categorization of subject's clinical parameter.

The term "comprises" or "comprising" or "contains" or "containing" includes the embodiments of "consist" or "consisting".

Assays and methods according to the present invention based on the use of tpx or determining tpx specific antibodies allow to diagnose and, in addition, to differentiate tuberculosis infection. In addition, tpx is particularly useful in the diagnosis of pulmonary tuberculosis in endemic settings using preferably recombinant mycobacterial antigens based on tpx. In particular, tpx allows to differentiate between low treatment responders and fast treatment responders during treatment of TB. Thus, it is possible to stratify the treatment regimen as well as to predict the clinical outcome or determining the treatment course in an individual.

In this connection, the term "intensive phase of TB treatment" refers to, the two month intensive phase treatment with first line TB drugs in DOTS.

As used herein, the term "thiolperoxidase" or "tpx" refers to the mycobacterial thiolperoxidase. It is preferred that the mycobacterial thiolperoxidase is the thiolperoxidase of *Mycobacterium tuberculosis*. In particular, tpx is a peptide of Seq. ID No. 2 or fragments or homologs thereof. Fragments or homologs of mycobacterial thiolperoxidase, in particular of the thiolperoxidase of Seq. ID No. 2, refers to antigenic oligopeptides or polypeptides derived from the mycobacterium thiolperoxidase. While fragments concern parts of the peptide, homologs refer to thiolperoxidase or fragments of said thiolperoxidase derived from other mycobacterial sources or thiolperoxidase derived from other species having the same activity as the mycobaceterial thiolperoxidase according to the present invention. In particular, the tpx molecule is a tpx of tuberculosis database No. RV1932. The skilled person is well aware of suitable fragments of tpx.

As used herein, the term "latent" includes the latent status of TB infection (LTBI) as well as the cured status after successful treatment unless otherwise indicated.

In a preferred embodiment, the present invention relates to the mycobacterial thiolperoxidase for use in the diagnosis or determination of tuberculosis infection wherein the tpx is a peptide of Seq. ID No. 2 of fragments or homologs thereof, or a peptide encoded by Seq. ID No. 1 or homolog sequences thereof due to the degenerative code.

In another embodiment, the present invention relates to the use of the mycobacterial thiolperoxidase as defined herein for use in the differentiation between latent tuberculosis infection and active tuberculosis infection. The present inventors recognized that tpx represents a valuable tool for differentiating between and to monitor e.g. the transition from latent to active status accordingly. The mycobacterial thiolperoxidase is particularly useful in the differentiation between individuals being slow treatment responders and fast treatment responders, in particular, during the intensive phase of TB treatment.

Today the most effective known standard treatment regimen against TB is known under the name "Directly Observed Treatment Short Course" (DOTS) and consists of a four drug intensive phase of two months, followed by a two-drug continuation phase of four months. Today, the international union against tuberculosis and lung disease recommends that sputum smear or culture status are taken at the end of the two months intensive phase of treatment in order to monitor patient progress (Enarson DA, Management of tuberculosis: A guide for low income countries, 5th ed. Paris, 2000). Failure of sputum conversion at this time point imply a continuation of the intensive phase of anti TB treatment with four drugs and sputum culture with drug sensitive-testing to exclude drug resistance. However, a time period of more than two months before the first indication of treatment efficiency is long and has implications for individual patients and control programs. Moreover, these long time periods allows the bacteria to adapt to the treatment, frequently creating drug tolerant and drug resistant strains during infective therapy. However, a timely identification of those patients who show a slow intensive treatment response would be of great benefit for the control of TB. Until today, no convincing serodiagnostic data are provided demonstrating a possibility to diagnose and differentiate between fast and slow intensive TB treatment responders. The present inventors aim to demonstrate that tpx represents a suitable tool for doing so. As demonstrated in the examples, determining the level or amount of antibodies against mycobacterial thiolperoxidase of an individual before intensive phase TB treatment enables to differentiate between possible slow treatment responders and fast treatment responders, thus, giving the artisan the possibility to adapt and configure the therapeutic regimen accordingly.

Hence, in another aspect, the present invention relates to a method for diagnosing or determining the status of tuberculosis infection in an individual afflicted with or suspected to be afflicted with tuberculosis infection, comprising:
a) determining the level or amount of antibodies against mycobacterium thiolperoxidase as a sample of an individual; and
b) determining or diagnosing the status of said individual based on the level or amount of antibodies against mycobacterium thiolperoxidase.

Another embodiment of the present invention relates to a method for the stratification of a therapeutic regimen of an individual afflicted with tuberculosis infection, in particular, during the intensive phase of TB treatment, comprising the steps of:
a) determining the level or amount of antibodies against mycobacterial thiolperoxidase, in particular, as defined herein a sample of said individual; and
b) determining the status of tuberculosis infection based on the level or amount of antibodies against said thiolperoxidase allowing differentiation of a latent and active status of tuberculosis infection in said individual or allowing differentiation of slow treatment responder and fast treatment responder of individuals being treated for tuberculosis infection.

As demonstrated herein, the serodiagnostic marker reflects significant differences between fast and slow early TB treatment response, thus, giving the possibility to adapt and configure the therapeutic regimen accordingly. Moreover, it is possible to use the serodiagnostic marker prospectively before start of the treatment.

In another embodiment, a method for predicting a clinical outcome or determining the treatment course in an individual afflicted with tuberculosis infection, in particular, during the intensive phase of TB treatment, is provided. Said method comprises the steps of:
a) determining the level or amount of antibodies against mycobacterial thiolperoxidase, in particular, as defined herein in a sample of said individual; and
b) determining the status of tuberculosis infection based on the level or amount of antibodies against said thiolperoxidase allowing differentiation of a latent and active status of tuberculosis infection in said individual or allowing differentiation of slow treatment responder and fast treatment responder of individuals being treated for tuberculosis infection.

Moreover, a method for monitoring the change from latent to active status of tuberculosis infection or vice versa afflicted with tuberculosis infection is provided. Said method comprises:
a) determining the level or amount of antibodies against mycobacterial thiolperoxidase, in particular, a thiolperoxidase as defined herein, in a sample of said individual; and
b) predicting the clinical outcome or determining the treatment course based on the level or amount of antibodies against said thiolperoxidase.

In the context of the present invention, the term "reference value" refers to an index value, a value derived from one or more computer indices, a value derived from a subject with known active or latent tuberculosis infection or a negative control, within a cohort of the same. In particular, the reference values obtained from individuals afflicted with latent and active status tuberculosis infections or fast and slow responders, and, in addition, the reference value represents a range or index obtained from at least two samples collected from individuals of said seize state.

Preferably, the sample of the individual to be tested is a blood sample or other body fluids including broncho-alveolar lavage and urine as well as tissues. For determining the presence of antibodies against tpx typically blood samples will be used.

Furthermore, tpx is suitable as an antigen in cell based tests, like cytokine release assays, e.g. IGRA or IL-2 based release assays. These cell based assays and suitable methods allowing for diagnosing or detecting the status of tuberculosis infection in an individual afflicted with or suspected to be afflicted with tuberculosis infection, as well as for the stratification of the therapeutic regimen, determining the treatment course or predicting the clinical outcome. In addition, suitable kits for use in the cell based assays using tpx as antigen for stimulation of said cells are provided. The skilled person is well aware of suitable assays known in the art using tpx as antigen substituting the antigen used in said assays before. In addition, the skilled person is in a position to determine the amount or concentration of the antigen in said cell based assays for cytokine release, e.g. from mononuclear cells.

In case of including the determination of the level or amount of cytokines released or produced from mononuclear cells after stimulation, whereby said mononuclear cells are obtained from the individual, the mononuclear cells are preferably obtained from blood and isolated by common techniques. Determination of the level or amount of cytokines may be affected both on protein or nucleic acid level. The skilled person is well aware of suitable methods useful for obtaining mononuclear cells from the individual as well as for determining cytokine level or amount.

Typically, the level or amount of antibodies is determined by an immunoassay, like ELISpot or ELISA. The same holds true for the determination of cytokines released in cell based assays, e.g. released from mononuclear cells after stimulation, namely stimulation with tpx. Of course, cytokines can also be measured by determining the mRNA levels corresponding to said cytokines, accordingly. The skilled person is well aware of suitable methods for determining the same nucleic acid level including PCR techniques.

As demonstrated herein, immunoassays determining antibodies against tpx, in a preferred embodiment in combination with determining antibodies against other mycobacterium proteins, allow to differentiate between fast and slow TB treatment responders. In addition, as demonstrated herein, differentiation between TB infection and non-infection and also between latent and acute TB infection is possible.

In a preferred embodiment, the methods according to the present invention comprise determining the level or amount of antibodies in the mycobacterial ESAT-6 and/or mycobacterial alanine dehydrogenase (AlaDH). In particular, with respect to the method of identifying slow responders and fast responders of individuals being treated for tuberculosis infection, in particular, intense phase TB treatment.

Slow responders demonstrate antibody levels against tpx and, preferably, in addition, against AlaDH and/or ESAT-6, fast responders show no signal or lower signals compared to the low responders. The titer of anti-tpx and, optionally, in addition, of anti AlaDH as well as anti ESA-6 in elevated with slow responders at the time point of diagnosis, i.e. before the start of treatment: hence, in contrast to the prior art which requires awaiting the first phase of TB treatment, the methods according to the present invention allows to predict responsiveness to drug treatment before start of the treatment.

In a preferred embodiment, the antibodies to be determined are anti-tpx IgG, anti-ESAT-6 IgA and/or anti-AlaDH IgG.

In another preferred embodiment, the methods according to the present invention include in addition the determination of the level or amount of antibodies against mycobacterial LAM and/or mycobacterial Apa.

In particular, the combination of determining the level or amount of antibodies against tpx with determining the level or amount of antibodies against mycobacterial LAM and/or mycobacterial Apa allows to differentiate between latent and acute status of tuberculosis infection. That is, a tool for serodiagnosis of latent and acute status of TB infection is provided based on determining antibodies against tpx, preferably, in combination with LAM and/or Apa.

However, it is preferred that IgG and/or IgA antibodies against the thiolperoxidase, in particular, antibodies are determined. Furthermore, it is preferred that ESAT-6 IgA and AlaDH igG are determined.

In case of LAM anti LAM IgG and/or anti LAM IgA are determined while for Apa anti Apa IgM is preferred.

When monitoring the change from latent to active status of tuberculosis infection or vice versa in an individual, the first sample from the individual is preferably obtained at a first time point while the second sample obtained after a, preferably predetermined, time point e.g. after undergoing treatment of tuberculosis infection or undergoing treatment for autoimmune diseases. For example, in case of the treatment of autoimmune diseases or other diseases whereby the immune system is modulated, it is helpful to determine the status of TB infection to avoid any onset of the TB infection. It is known when treatment of RA patients, latent status TB infection may proceed to active status. That is, the present invention allows to determine the treatment course in said individual or predicting the clinical outcome by identifying the actual status of tuberculosis infection in said individual. Furthermore, it is possible to differentiate between non-infected individuals and individuals infected with TB in latent status due to elevated levels of antibodies against tpx against LAM and/or APA.

Important clinical implications arise from the finding described herein. Due to the possibility to differentiate between non-infected and latent effected individuals as well as differentiating between latent and active status it is possible to stratify the therapeutic regimen or determine the treatment course in the individual accordingly. The same is true for the possibility to differentiate between high and low responding individuals for intensive TB treatment before starting therapy.

In a further aspect, the present invention relates to the use of a kit for diagnosing or determining the status of tuberculosis infection, or for predicting a clinical outcome or determining the treatment course in an individual afflicted with or suspected to be afflicted with tuberculosis infection, or for the stratification of the therapeutic regimen of an individual with tuberculosis infection or for monitoring the progression of tuberculosis infection, or for determining the transition from latent to active status of tuberculosis infection in an individual, said kit comprises means for determining the level or amount of antibodies against mycobacterial thiolperoxidase, optionally, means for determining the level or amount of antibodies against ESAT-6 and/or AlaDH and/or LAM and/or Apa, and instructions on how to use said test kit for a method according to the present invention.

Preferably, the kit contains additionally instructions on how to use the test kit for a method according to the present invention. In particular, in a preferred embodiment said kit is an immunoassay, like an ELISpot or ELISA, for determining the level or amount of antibodies against one of the mycobacterial antigens mentioned herein. Alternatively, in case of cell based assays, the kit is an ELISpot or ELISA or other suitable immunoassay for determining the amount of the cytokine, accordingly. Preferably, cytokines are interferon gamma and/or interleukin-2. In another embodiment, the kit is a PCR-kit with means suitable for analyzing a cytokine expression on nucleic acid level.

Moreover, the present invention relates to an assay for conducting the method according to the present invention comprising means for determining the level or amount of antibodies against mycobacterial tpx, in particular, tpx of mycobacterium tuberculosis. In addition, said assay may further contain means for determining the level or amount of antibodies against any one of the additional mycobacterial antigen as mentioned herein.

Finally, the present invention relates to cell based assays and methods wherein tpx is the antigen for stimulating cytokine release from the cells derived from the individual to be tested. Typically, an individual afflicted with or suspected to be afflicted with tuberculosis infection.

The above disclosure generally describes the present invention. More complete understanding can be obtained by reference to the following specific examples of embodiments of the invention without being limited thereto.

### Example 1

### Serodiagnostical markers for differentiating between slow and fast responders

21 HIV-unaffaceted pulmonary, smear positive, first time TB patients have been tested. In brief, all TB patients were cured after six months of DOTS treatment and after the two months intensive phase of treatment, 15 out of 21 patients were smear-negative but only 8 were culture-negative. With conventional techniques, namely the month 2 BACTEC culture was used as more sensitive indicator for a treatment response. The 8 patients who showed sputum conversion at month 2 were called fast responders while the 13 patients who remained sputum culture positive were called slow responders. No significant differences between fast and slow responders in CXR findings at diagnosis were found. As controls 17 healthy TSPOT.TB-negative and QuantiFERON TB Gold in tube negative community controls complemented by a further 3 healthy community controls have been used.

Titers of IgG and/or IgA antibodies against TPX (IgG), ESAT-6 (IgA) and AlaDH (IgG) have been used. Measurement of the antibodies was performed by ELISA using purified antigens. Detection of antibodies against a specific antigen was done using anti human IgG or anti human IgA or anti human IgM antibodies labelled with horseradish perodixdase (HRP) obtained from Pierce. ELISA was performed according to standard techniques.

In figure 1 box-and whisker plots showing the optical density (OD) values of the anti ESAT-6 IgA, anti TPX IgG and anti AlaDH serodiagnosis assays for the comparison of 8 fast and 13 slow intensive anti TB treatment responders. As clearly identified, the anti ESAT-6, anti TPX and anti AlaDH antibodies are higher in slow responders compared to fast responders, thus, allow differentiation between these two types. This is demonstrated in more detail in table 1

**Table 1**

| Specificities and sensitivities of individual seroantigens with sera from 21 | | | |
|---|---|---|---|
| South-African tuberculosis patients (including eight fast and 13 slow treatment responders) and 20 healthy community controls | | | |
| Antigen | Ig class | Sensitivity (%) based ity on 95 % specificity | |
| | | fast responders | slow responders |
| Tpx | IgG | 0.0 | 53.8 |
| ESAT-6 | IgA | 0.0 | 53.8 |
| AlaDH | IgG | 0.0 | 38.5 |

As shown in Table 1, the three antigens Tpx, AlaDH and ESAT-6 did exclusively select slow responders whereas fast responders show no signal above the cut-off.

Moreover, as shown in table 2 the levels of anti Tpx IgG and anti ESAT-6 IgA antibodies were able to differentiate with 100 % specificity and 84,6 % sensitivity and hence an accuracy of 90,5 % between 8 fast responders and 13 slow responders as defined by their month 2 sputum culture status, respectively. Hence, it is possible to differentiate between fast and slow responders even prior to initiation of therapy. That is, it is possible to identify slow responders to early treatment prospectively. As demonstrated in, anti Tpx IG preferably in combination with anti ESAT-6 IgA antibodies measured directly before initiation of therapy predicted on two month positive sputum cultures with high sensitivity at very high specificity. By combining anti AlaDH IgG levels, the prognosis can be increased further. Hence, it is possible to use serodiagnostic markers as predictors for high-risk patients (slow responders),thus, to improve treatment efficacy and to expedite anti-TB drug trials.

**Table 2**

| Percentage of correct prediction of 13 slow and 8 fast intensive phase tuberculosis treatment responders using discriminant analysis at diagnosis | | | |
|---|---|---|---|
| | predicted as fast responders | predicted as slow responders | % correct prediction^{#} |
| Fast responders | 8 | 0 | 100 (specificity) |
| Slow responders | 2 | 11 | 84.6 (sensitivity) |

| | | | |
|---|---|---|---|
| ^{#} variables chosen = anti-Tpx IgG, anti-ESAT-6 IgA; leave-one-out crossvalidated | | | |

### Example 2

### Serodiagnosis of latent tuberculosis infection

27 apparently healthy individuals with no clinical signs of TB or other diseases have been enrolled in the study. Blood samples were collected from the participants and commercial IGRAs were performed. The healthy controlled individuals received BCG vaccination routinely at birth. 47 out of the 67 apparently healthy individuals showed QFT and/or TSPOT.TB positive results, thus, being grouped into the latent *M-tuberculosis* infected subjects (LTBI group).

By ELISA it was demonstrated that latently TB infected individuals compared to healthy individuals show higher Tpx levels.

### Example 3

Serodiagnosis of pulmonary tuberculosis in endemic settings, in particular, with respect to latent and active TB infection.

The 47 persons identified in example 2 as representing LTBI subjects were compared with 42 individuals suffering from active TB infection. As shown in Table 3, the antigen according to the present invention is suitable to differentiate between active TB and latent TB. In particular, in combination with the mycobacterial antigens LAM and/or APA differentiation between latent and active status is possible. In particular, table 3 demonstrates that when the antibodies are anti-px IgG and anti-Tpx IgA and/or anti LAM IgG and/or anti LAM IgA and/or anti-Apa IgM, differentiation discrimination between LTBI and TB disease is possible with high sensitivity and specificity.

**Table 3**

| Accuracies of seroantigen combinations to distinguish between TB disease (n=42) and LTBI (n=47) after general discriminant analysis | | | | | | |
|---|---|---|---|---|---|---|
| Antigen combination | Resubstitution classification matrix | | | Leave-one-out cross-validation | | |
| | % of LTBI correctly classified | % of TB cases correctly classified | Accuracy % | % of LTBI correctly classified | % of TB cases correctly classified | Accuracy % |
| LAM-IgA, | 91.5 | 81.0 | 86.5 | 91.5 | 81.0 | 86.5 |
| LAM-IgG, | | | | | | |
| tpx-IgA, tpx- | 43/47 | 34/42 | | 43/47 | 34/42 | |
| IgG, Apa- | | | | | | |
| IgM* | | | | | | |

As demonstrated, a serodiagnostic test based on determining anti Tpx antibodies, preferably in combination with anti LAM and/or anti Apa antibodies allow to provide a TB diagnostic test to differentiate between latent and active status, thus, providing a highly sensitive and specific and also cost effective test for classifying LTB subjects and TB patients, respectively.

## Claims

1. Mycobacterial thiolperoxidase, in particular, thiolperoxidase of *Mycobacterium tuberculosis*, for use in diagnosing or determining tuberculosis infection in an individual.

2. Mycobacterial thiolperoxidase for use in the diagnosis or determination of tuberculosis infection in an individual according to claim 1 wherein the thiolperioxidase (tpx) is a peptide of SEQ ID No. 2 or fragments or homologs thereof or a peptide encoded by SEQ ID No. 1 or homologs of SEQ ID No. 1.

3. The use of a mycobacterial thiolperoxidase as defined in any one of claims 1 or 2 for use in the differentiation between individuals being slow treatment responders and fast treatment responders, in particular, of fast treatment responders and slow treatment responders of the intensive phase of TB treatment.

4. The use of a mycobacterial thiolperoxidase as defined in any one of claims 1 or 2 for use in the differentiation between latent tuberculosis infection and active tuberculosis infection.

5. A method for the stratification of a therapeutic regimen of an individual afflicted with tuberculosis infection, in particular, for the intensive phase of TB treatment comprising:
a) determining the level or amount of antibodies against mycobacterial thiolperoxidase, in particular, as defined in one of claims 1 or 2 in a sample of said individual; and
b) determining the status of tuberculosis infection based on the level or amount of antibodies against said thiolperoxidase allowing differentiation of a latent and active status of tuberculosis infection in said individual or allowing differentiation of slow treatment responder and fast treatment responder of individuals being treated for tuberculosis infection.

6. A method for predicting a clinical outcome or determining the treatment course or the status of tuberculosis infection in an individual afflicted with tuberculosis infection comprising:
a) determining the level or amount of antibodies against mycobacterial thiolperoxidase, in particular, a thiolperoxidase as defined in any one of claims 1 or 2, in a sample of said individual; and
b) predicting the clinical outcome or determining the treatment course based on the level or amount of antibodies against said thiolperoxidase.

7. A method for monitoring the change from latent to active status of tuberculosis infection or vice versa in an individual afflicted with tuberculosis infection comprising:
a) determining the level or amount of antibodies against mycobacterial thiolperoxidase, in particular, thiolperoxidase as defined in one of claims 1 or 2, in a sample of said individual at a first point of time;
b) determining the level or amount of antibodies against mycobacterial thiolperoxidase, in particular, as defined in any one of claims 1 or 2, in a sample obtained from said individual at a second point of time; and
c) comparing the level or amount of antibodies determined in step a) to the level or amount determined in step b) or to a reference value whereby an increase in the level or in the amount relative to a reference value or to the level or amount determined in step a) is indicative for a transition from latent to active status in a decrease in the level or the amount relative to a reference value or to the level or amount determined in step a) is indicative for a transition from active to latent status.

8. The method according to any one of claims 5 to 7 wherein the sample of the individual is a blood sample or other body fluids including Bronchoalveolar lavage and urine as well as tissues.

9. The method according to any one of claims 5 to 8 wherein the level or amount of antibodies is determined by an immuno assay, like ELISpot or ELISA.

10. The method according to any one of claims 5 to 6, 8 or 9 further comprising determining the level or amount of antibodies against the mycobacterial ESAT-6 and/or mycobacterial Alanine Dehydrogenase (AlaDH), preferably the antibodies to be determined are anti-tbx IgG, anti-ESAT-6 IgA and/or anti-AlaDH IgG.

11. The method according to any one of claims 5 to 9 further comprising determining the level or amount of antibodies against mycobacterial LAM and/or mycobacterial Apa, preferably wherein the antibodies are anti-tbx IgG and/or anti-tbx IgA and/or anti-LAM IgG and/or anti-LAM IgA and/or anti-Apa IgM.

12. The method according to any one of claims 5 to 11 wherein IgG and/or IgA antibodies against the thiolperoxidase, preferably, IgG antibodies are determined.

13. The use of a kit for diagnosing or determining the status of tuberculosis infection, or for predicting a clinical outcome or determining the treatment course in an individual afflicted with or suspected to be afflicted with tuberculosis infection, or for the stratification of the therapeutic regimen of an individual with tuberculosis infection or for monitoring the progression of tuberculosis infection, or for determining the transition from latent to active status of tuberculosis infection in an individual, said kit comprises means for determining the level or amount of antibodies against mycobacterial thiolperoxidase, optionally, means for determining the level or amount of antibodies against ESAT-6 and/or AlaDH and/or LAM and/or Apa, and instructions on how to use said test kit for a method according to any one of claims 5 to 12.

14. The use of a kit according to claim 13 whereby said kit is at least ELISpot or ELISA.

15. An assay for conducting the method according to any one of claims 5 to 12 comprising means for determining the level or amount of antibodies against mycobacterial tpx, in particular, tpx of mycobacterium tuberculosis.


SEQUENCE LISTING

<110>  Lionex Diagnostics and Therapeutics GmbH

<120>  Mycobacterial thiolperoxidase and its use

<130>  3441-010-EP-1

<160>  2

<170>  PatentIn version 3.5

<210>  1
<211>  498
<212>  DNA
<213>  Mycobacterium tuberculosis


<220>
<221>  CDS
<222>  (1)..(498)

<400>  1
atg gca cag ata acc ctg cga gga aac gcg atc aat acc gtc ggt gag      48
Met Ala Gln Ile Thr Leu Arg Gly Asn Ala Ile Asn Thr Val Gly Glu
1               5                   10                  15

cta cct gct gtc gga tcc ccg gcc ccg gcc ttc acc ctg acc ggg ggc      96
Leu Pro Ala Val Gly Ser Pro Ala Pro Ala Phe Thr Leu Thr Gly Gly
            20                  25                  30

gat ctg ggg gtg atc agc agc gac cag ttc cgg ggt aag tcc gtg ttg      144
Asp Leu Gly Val Ile Ser Ser Asp Gln Phe Arg Gly Lys Ser Val Leu
        35                  40                  45

ctg aac atc ttt cca tcc gtg gac aca ccg gtg tgc gcg acg agt gtg      192
Leu Asn Ile Phe Pro Ser Val Asp Thr Pro Val Cys Ala Thr Ser Val
    50                  55                  60

cga acc ttc gac gag cgt gcg gcg gca agt ggc gct acc gtg ctg tgt      240
Arg Thr Phe Asp Glu Arg Ala Ala Ala Ser Gly Ala Thr Val Leu Cys
65                  70                  75                  80

gtc tcg aag gat ctg ccg ttc gcc cag aag cgc ttc tgc ggc gcc gag      288
Val Ser Lys Asp Leu Pro Phe Ala Gln Lys Arg Phe Cys Gly Ala Glu
                85                  90                  95

ggc acc gaa aac gtc atg ccc gcg tcg gca ttc cgg gac agc ttc ggc      336
Gly Thr Glu Asn Val Met Pro Ala Ser Ala Phe Arg Asp Ser Phe Gly
            100                 105                 110

gag gat tac ggc gtg acc atc gcc gac ggg ccg atg gcc ggg ctg ctc      384
Glu Asp Tyr Gly Val Thr Ile Ala Asp Gly Pro Met Ala Gly Leu Leu
        115                 120                 125

gcc cgc gca atc gtg gtg atc ggc gcg gac ggc aac gtc gcc tac acg      432
Ala Arg Ala Ile Val Val Ile Gly Ala Asp Gly Asn Val Ala Tyr Thr
    130                 135                 140

gaa ttg gtg ccg gaa atc gcg caa gaa ccc aac tac gaa gcg gcg ctg      480
Glu Leu Val Pro Glu Ile Ala Gln Glu Pro Asn Tyr Glu Ala Ala Leu
145                 150                 155                 160

gcc gcg ctg ggc gcc tag                                              498
Ala Ala Leu Gly Ala
                165

<210>  2
<211>  165
<212>  PRT
<213>  Mycobacterium tuberculosis

<400>  2

Met Ala Gln Ile Thr Leu Arg Gly Asn Ala Ile Asn Thr Val Gly Glu
1               5                   10                  15

Leu Pro Ala Val Gly Ser Pro Ala Pro Ala Phe Thr Leu Thr Gly Gly
            20                  25                  30

Asp Leu Gly Val Ile Ser Ser Asp Gln Phe Arg Gly Lys Ser Val Leu
        35                  40                  45

Leu Asn Ile Phe Pro Ser Val Asp Thr Pro Val Cys Ala Thr Ser Val
    50                  55                  60

Arg Thr Phe Asp Glu Arg Ala Ala Ala Ser Gly Ala Thr Val Leu Cys
65                  70                  75                  80

Val Ser Lys Asp Leu Pro Phe Ala Gln Lys Arg Phe Cys Gly Ala Glu
                85                  90                  95

Gly Thr Glu Asn Val Met Pro Ala Ser Ala Phe Arg Asp Ser Phe Gly
            100                 105                 110

Glu Asp Tyr Gly Val Thr Ile Ala Asp Gly Pro Met Ala Gly Leu Leu
        115                 120                 125

Ala Arg Ala Ile Val Val Ile Gly Ala Asp Gly Asn Val Ala Tyr Thr
    130                 135                 140

Glu Leu Val Pro Glu Ile Ala Gln Glu Pro Asn Tyr Glu Ala Ala Leu
145                 150                 155                 160

Ala Ala Leu Gly Ala
                165